**Europäisches Patentamt**

**European Patent Office**

**Office européen des brevets**

(19)

(11) Veröffentlichungsnummer: **0 152 842 B1**

(12) **EUROPÄISCHE PATENTSCHRIFT**

(45) Veröffentlichungstag der Patentschrift: **26.08.87**

(21) Anmeldenummer: **85101051.2**

(22) Anmeldetag: **01.02.85**

(51) Int. Cl.⁴: **C 07 C 39/08, C 07 C 37/07**

(54) **Verfahren zur Herstellung von Trimethylhydrochinon.**

(30) Priorität: **08.02.84 DE 3404337**

(43) Veröffentlichungstag der Anmeldung:
**28.08.85 Patentblatt 85/35**

(45) Bekanntmachung des Hinweises auf die Patenterteilung:
**26.08.87 Patentblatt 87/35**

(84) Benannte Vertragsstaaten:
**CH DE FR GB LI NL**

(56) Entgegenhaltungen:
**DE - B - 1 940 386**
**FR - A - 2 341 551**

(73) Patentinhaber: **BASF Aktiengesellschaft,
Carl-Bosch-Strasse 38, D-6700 Ludwigshafen (DE)**

(72) Erfinder: **Broecker, Franz Josef, Dr., Schwanthaler
Allee 20, D-6700 Ludwigshafen (DE)**
Erfinder: **Tavs, Peter, Dr., Neuhofener Strasse 45,
D-6703 Limburgerhof (DE)**
Erfinder: **Laas, Harald, Dr., Woehlerstrasse 14,
D-6701 Maxdorf (DE)**
Erfinder: **Aders, Wolf-Karlo, Dr., Haardtstrasse 40,
D-6701 Ellerstadt (DE)**

## Beschreibung

Die Erfindung betrifft ein Verfahren zur kontinuierlichen Herstellung von hochreinem 2,3,6-Trimethylhydrochinon durch katalytische Hydrierung mit Wasserstoff in Gegenwart eines Lösungsmittels an einem Platin-Aluminiumoxid-Katalysator.

2,3,6-Trimethylhydrochinon ist ein Zwischenprodukt zur Herstellung von Vitamin E. Es sind bereits Methoden zur Herstellung von 2,3,6-Trimethylhydrochinon bekannt. So lassen sich Chinone üblicherweise mit Eisenpulver in schwefelsaurer Lösung oder mit Natriumhydrogensulfat zu Hydrochinonen reduzieren. Dabei fallen unerwünschte Sulfatlösungen als Nebenprodukte an, die diese Verfahren unwirtschaftlich machen. Bekannt ist ferner ein Verfahren zur Herstellung von Trimethylhydrochinon durch katalytische Reduktion von Trimethylchinon mit Wasserstoff in Gegenwart eines Hydrierkatalysators, dadurch gekennzeichnet, dass man die Umsetzung mit aliphatischen Alkoholen mit 3 bis 5 C-Atomen als Lösungsmittel durchführt.

Bei einer kontinuierlichen Arbeitsweise wie sie in DT-OS 1 940 386 beschrieben ist, reichern sich die Nebenprodukte bei der Rückführung des als Lösungsmittel verwendeten Alkohols ständig an, so dass ein Teil der Mutterlauge ausgeschleust werden muss. Das führt zu Verlusten an Wertprodukt.

Auch das in der DT-OS 2 250 066 beschriebene Verfahren zur Herstellung von 2,3,5-Trimethylhydrochinon durch katalytische Hydrierung von 2,3,5-Trimethylbenzochinon mit Wasserstoff in einem organischen Carbonsäureester als Lösungsmittel liefert erst, nach einer grosstechnisch nur mit erheblichen Kosten zu realisierenden Fällung, ein entsprechend reines Produkt.

Es ist ferner ein Verfahren zur Herstellung von Hydrochinonen durch Reduktion der Chinone mit Wasserstoff in Gegenwart eines Kupferchromitkatalysators bei Drücken von 10 bis 300 bar und Temperaturen von 20 bis 300°C in der DT-OS 1 956 381 beschrieben. Auch bei diesem Verfahren erhält man Hydrochinone ungenügender Reinheit, so dass das Hydrierprodukt nach dem Abdampfen des Lösungsmittels noch umkristallisiert werden muss, um die erforderliche Reinheit zu erhalten. Ausserdem muss ein Stabilisator dem so gewonnenen Hydrochinon zugesetzt werden, um eine Verfärbung zu vermeiden.

Es bestand deshalb die Aufgabe, ein einfache Hydrierverfahren zu entwickeln, bei dem die Nebenproduktbildung bei der Hydrierung unterbunden wird und damit die beschriebenen Reinigungsmassnahmen am Hydrierprodukt überflüssig werden.

Es wurde nun gefunden, dass man kontinuierlich 2,3,6-Trimethylhydrochinon durch katalytische Hydrierung von 2,3,6-Trimethylchinon mit Wasserstoff in Gegenwart eines Lösungsmittels herstellen kann, wenn man die Umsetzung an einem Platin/Aluminiumoxid-Katalysator welcher die im Patentanspruch 1 angegebene Charakteristik aufweist, durchführt.

Aufgrund der ausgezeichneten Selektivität dieses Katalysators kann das Lösungsmittel durch einfaches Abdampfen vom Hydrieraustrag abgetrennt werden und man erhält 2,3,6-Trimethylhydrochinon, welches keiner weiteren Reinigung unterzogen werden muss. Grundlage des erfindungsgemässen Verfahrens ist ein Katalysator, der eine 100%ige Selektivität besitzt und daher keinerlei Nebenprodukte bildet. Dadurch wird ein kontinuierlich arbeitendes Verfahren ermöglicht, bei dem durch einfaches Abdampfen des Lösungsmittels hochreines, kristallines 2,3,6-Trimethylhydrochinon gewonnen wird, welches sich bei längerer Lagerung nicht verfärbt. Durch die Einsparung der aufwendigen Reinigungsverfahren wird die technische Hydrochinonherstellung erheblich verbessert. Im Gegensatz zu den bekannten Verfahren, bei denen durch das Ausschleusen der Nebenprodukte stets auch Wertprodukt verloren gehen, tritt beim erfindungsgemässen Verfahren kein Wertproduktverlust auf. Die Katalysatordesaktivierung wird vermieden, so dass man bei dem erfindungsgemässen Verfahren auch lange Katalysatorstandzeiten erhält.

Bei der Herstellung des erfindungsgemässen Pt/Al$_2$O$_3$-Katalysators kommt es auf eine bestimmte spezifische Oberfläche nach BET und eine definierte Porenverteilung an. Der erfindungsgemässe Katalysator basiert auf einem Träger aus Aluminiumoxid, vorzugsweise γ-Al$_2$O$_3$, mit einer BET-Oberfläche von 1 bis 350, vorzugsweise 10 bis 300, insbesondere 200 bis 270 m$^2$/g und einem Porenvolumen im Porenradienbereich zwischen 1,8 und 60.000 nm von 0,35 bis 0,60 cm$^3$/g, insbesondere 0,45 bis 0,55 cm$^3$/g, mit der Massgabe, dass mindestens 60% des Porenvolumens, vorzugsweise 80 bis 90%, im Bereich von 2 bis 200 nm, vorzugsweise 2 bis 100 nm Porenradius liegen.

Zur Herstellung dieses speziellen Trägermaterials wird Aluminiumhydroxid Al(OH)$_3$ gefällt, getrocknet, durch Verpressen oder Verstrangen in Formlinge umgewandelt und anschliessend bei Temperaturen von 300 bis 1200°C, vorzugsweise 400 bis 1150, insbesondere 400 bis 600°C kalziniert. Man führt diese Kalzination so durch, dass ein Trägermaterial entsteht, das durch die oben angegebenen physikalischen Daten charakterisiert wird.

Der so erhaltene Träger wird dann mit einer wässrigen Platinnitratlösung getränkt, so dass ein Katalysator mit 0,01 bis 0,5, vorzugsweise 0,05 bis 0,15 Gew.-% Pt gewonnen wird. Zum Tränken des Trägermaterials wird die Lösungsmenge so bemessen, dass sie 90% der Wasseraufnahmekapazität des Trägers entspricht. Mit dieser Lösungsmenge wird der Träger, beispielsweise in einer Dagiertrommel, getränkt und anschliessend bei 100 bis 150°C, vorzugsweise 110 bis 130°C getrocknet und danach bei 180 bis 250°C kalziniert. Der so erhaltene Katalysator wird in einen Festbetthydrierreaktor eingebaut und 0,5 bis 6 h bei 50 bis 200°C, vorzugsweise 100°C, mit Wasserstoff reduziert. Anschliessend wird bei 80 bis 110°C, vorzugsweise 95 bis 105°C und einem Wasserstoffpartialdruck von 1 bar eine Lösung von 2,3,6-Trimethylchinon in Isobutylalkohol über den Katalysator geleitet. Das Hydrierprodukt wird im Rotationsverdampfer vom Lösungsmittel befreit und analysiert. Die Belastung des Katalysators bei dieser Hydrierung beträgt 0,05 bis 0,6 [kg TMC/1$_{Kat.}$-h], insbesondere 0,1 bis 0,25 [kg TMC/$_{Kat.}$ · H].

Die Hydrierung kann in Sumpf- oder Rieselfahrweise mit Alkoholen als besonders geeigneten Lösungsmitteln durchgeführt werden. Bei der Rieselfahrweise wird zweckmässig mit einer Querschnittsbelastung von 20 bis 80 $[\frac{m^3}{m^2 \cdot h}]$ gearbeitet. Dabei liegt das Rücklaufverhältnis (Rv = $\frac{\text{Masse Rücklauf}}{\text{Masse Zulauf}}$) zwischen 10 und 65. Die Katalysatorbelastung, bezogen auf TMC, beträgt etwa 0,05 bis 0,6 [kg TMC/1$_{Kat.}$·h]. Mittels eines Wärmeaustauschers im Hydrierkreislauf kann man die Reaktoreintrittstemperatur zwischen 80 und 120°C einstellen. Der Wasserstoffpartialdruck bei der TMC-Hydrierung liegt etwa bei 0,5 bis 5 bar. Zur Vermeidung einer Inertenanreicherung kann man eine bestimmte Menge Abgas entnehmen. Die Abgasmenge wird zweckmässig so bemessen, dass der Inertengehalt 5 bis 10 Vol.-% nicht überschreitet.

*Beispiel 1*

Aus einer Aluminiumnitratlösung wird mit verdünnter Natronlauge Hydrargillit Al(OH)$_3$ ausgefällt, gewaschen und bei 100 bis 150°C getrocknet. Das Trockenprodukt wird in einer Kugelmühle gemahlen und anschliessend in einem Kneter mit Wasser zu einer Paste verarbeitet. Diese Paste wird mit einer Strangpresse zu 3 mm-Strängen verarbeitet. Die Stränge werden bei 120°C getrocknet und anschliessend 5 h bei 450°C in einem Muffelofen kalziniert. Man erhält ein γ-Al$_2$O$_3$ mit einer BET-Oberfläche von 254 m²/g, einem Porenvolumen im Bereich zwischen 1,8 und 60.000 nm Porenradius von 0,521 [cm³/g], wobei 85% des Porenvolumens im Porenradienbereich zwischen 2 und 100 nm liegen.

1 kg dieses Trägermaterials, in Form von 3 mm-Strängen, wird in einer Dragiertrommel mit einer wässrigen Lösung von 1,79 g Platin-II-nitrat (55,87 Gew.-% Pt) der Fa. Heraeus in 610 ml Wasser versetzt. Die Lösungsmenge entspricht etwa 90% der Wasseraufnahmekapazität des Trägers. Träger und Lösung werden 15 bis 20 Minuten in der Dragiertrommel bewegt, so dass die Flüssigkeit gleichmässig vom Trägermaterial aufgenommen wird. Der feuchte Katalysator wird anschliessend 16 Stunden bei 120°C getrocknet und danach 8 Stunden bei 200°C kalziniert. Der Katalysator enthält 0,1 Gew.-% Platin.

100 ml dieses Katalysators werden in einen Hydrierreaktor eingebaut und unter Stickstoff auf eine Temperatur von 100°C aufgeheizt. Nach Erreichen der Temperatur wird der Stickstoff durch Wasserstoff ersetzt und der Katalysator mit H$_2$ 1 Stunde reduziert. Dann wird eine 20%ige Lösung von 2,3,6-Trimethylchinon in Isobutanol über den Katalysator geleitet und eine Abgasmenge von 1,2 l/h eingestellt. Die Temperatur wird auf 100°C gehalten. Die Belastung beträgt, bezogen auf 2,3,6-Trimethylchinon (TMC) 0,135 [kg TMC/1$_{Kat.}$·H]. Das Hydrierprodukt wird im Rotationsverdampfer vom Lösungsmittel befreit und analysiert. Der Umsatz, bezogen auf 2,3,6-Trimethylchinon, die Ausbeute und damit auch die Selektivität beträgt 100%. Das erhaltene weisse 2,3,6-Trimethylhydrochinon verändert seine Farbe bei der Lagerung nicht, da es von höchster Reinheit ist.

Zum Vergleich der erfindungsgemässen Katalysatoren mit anderen Katalysatoren wurden die nachfolgenden Versuche durchgeführt:

Versuch 1: Einfluss des Trägermaterials

Es wird ein Platinkatalysator mit Aktivkohle als Trägermaterial hergestellt. Dazu werden 400 g Aktivkohle, in Form von 4 mm-Strängen mit einer Lösung von 0,72 g Platin-II-nitrat (55,87 Gew.-% Pt) in 480 ml destilliertem Wasser versetzt und 10 Minuten in der Dragiertrommel bewegt. Der feuchte Katalysator wurde dann 18 h bei 120°C getrocknet und weitere 6 h bei 200°C erhitzt.

Analog dem Beispiel wird dieser Katalysator mit 0,1 Gew.-% Pt auf Aktivkohle reduziert und für die Hydrierung von 2,3,6-Trimethylchinon eingesetzt. Nach dem Abdampfen des Lösungsmittels wird das Hydrochinon analysiert. Es wurde folgendes Ergebnis erzielt:

| | |
|---|---|
| Umsatz: | 99,8% |
| Ausbeute: | 92,6% |
| Selektivität: | 92,8% |

Das Beispiel zeigt, dass eine beträchtliche Nebenproduktbildung erfolgt.

Versuch 2: Palladium als Aktivkomponente

Es wird ein handelsüblicher Palladiumkatalysator mit 0,5 Gew.-% Pd auf SiO$_2$ als Träger eingesetzt und analog dem Beispiel hydriert. Unter den angegebenen Standardbedingungen erhält man folgendes Ergebnis:

| | |
|---|---|
| Umsatz an TMC: | 98,6% |
| Ausbeute an TMCH: | 91,8% |
| Selektivität | 93,1% |

Versuch 3: Palladium als Aktivkomponente

Wie in Beispiel 1 beschrieben, werden Aluminiumoxidstränge (3 mm) hergestellt und anschliessend 7 h bei 1050°C getempert. Man erhält einen Träger mit 25 m²/g Oberfläche nach BET und einem Porenvolumen von 0,380 cm³/g im Porenradienbereich zwischen 1,8 und 60.000 nm, wobei 80% dieses Volumens zwischen 2 und 100 nm Porenradius liegen.

800 g dieses Trägers werden mit einer Lösung aus 36.6 g Palladiumnitratlösung (11 Gew.-%) und 310 ml destilliertem Wasser getränkt und 10 Minuten in der Dragiertrommel bewegt. Der feuchte Katalysator wird danach 7 h bei 120°C getrocknet und 12 h auf 200°C erhitzt. Man erhält so einen Katalysator mit 0,5 Gew.-% Pd auf Aluminiumoxid als Träger. 100 ml dieses Katalysators werden für die Hydrierung unter den im Beispiel beschriebenen Bedingungen verwendet. Man erhält:

| | |
|---|---|
| Umsatz an TMC: | 99,8% |
| Ausbeute an TMCH: | 92,7 |
| Selektivität: | 92,9 |

Die Versuche 2 und 3 zeigen, das Palladium erhebliche Nebenprodukte liefert, und zwar auch mit Al$_2$O$_3$ als Trägermaterial. Nur mit Platin als Aktivkomponente und Al$_2$O$_3$ als Trägermaterial ist eine 100%ige Selektivität erreichbar. Dieses Ergebnis ist überraschend, da nach DT-OS 1 956 381 Edelmetallträgerkatalysatoren nur sehr kurze Laufzeiten ergeben.

*Beispiel 2*

Aluminiumhydroxidpaste wird, wie in Beispiel 1 angegeben, hergestellt und zu Formlingen verpresst, getrocknet und anschliessend 5 h bei 1100°C kalziniert. Es entsteht $\alpha$-$Al_2O_3$ mit einer BET-Oberfläche von 12 m²/g und einem Porenvolumen im Porenradienbereich von 1,8 bis 60.000 nm von 0,520 cm³/g, wobei 70% des Porenvolumens im Radienbereich zwischen 2 und 100 nm liegen.

1,2 kg dieses Trägers werden in einer Dragiertrommel mit einer Lösung aus 2,17 g Platin-II-nitrat (55,22 Gew.-% Pt) in 500 ml destilliertem Wasser versetzt. Die Trommel wird 10 Minuten bewegt und der feuchte Katalysator dann 8 h bei 120°C getrocknet und anschliessend weitere 8 h bei 200°C erhitzt. Der so erhaltene Katalysator enthält 0,1 Gew.-% Pt auf $\alpha$-$Al_2O_3$.

100 ml dieses Katalysators werden analog Beispiel 1 reduziert und für die Hydrierung von 2,3,6-Trimethylchinon eingesetzt. Unter den in Beispiel 1 angegebenen Bedingungen und einer Belastung von 0,135 [kg TMC/1$_{Kat.}$ · h] erhält man bei der Hydrierung folgende Ergebnisse:

| | |
|---|---|
| Umsatz: | 98,4% |
| Ausbeute: | 98,4% |
| Selektivität: | 100 % |

Die Selektivität ist auch hier 100%, während der Umsatz aufgrund der kleineren BET-Oberfläche niedriger liegt als im Beispiel 1.

*Beispiel 3*

Für Versuche zur Katalysatoraktivität über längere Zeit wurde eine Versuchsapparatur zur kontinuierlichen Hydrierung verwendet. Die Apparatur besteht aus einem senkrecht stehenden Rohr (ca. 3,0 × 130 cm) als Hydrierreaktor, welches aussen beheizt ist. Unter dem Rohr befindet sich ein Abscheider von etwa 3 l.

Aus dem Abscheider wird Hydrierlösung im Kreis gepumpt. Am oberen Ende des Rohres werden frisches Lösungsmittel, 2,3,6-Trimethylchinon (TMC) und Wasserstoff zudosiert. Die Lösung läuft über das Katalysatorbett und sammelt sich im Abscheider. Aus dem Abscheider werden Abgas und Flüssigkeit kontinuierlich abgenommen.

In den Hydrierreaktor werden 755 g Katalysator — wie in Beispiel 1 beschrieben — eingefüllt und im Wasserstoffstrom bei gleichzeitigem Isobutanolkreislauf (50 l/h) reduziert. Alle nachfolgend beschriebenen Versuche werden mit einem Wasserstoffdruck von etwa 2 bar am oberen Ende des Hydrierreaktors und 50 ± 10 l Flüssigkeitskreislauf durchgeführt. Es werden zunächst 145 g TMC/h und 720 ml Isobutanol/h zudosiert. Der TMC-Zulauf wird dann in mehreren Stufen bis auf 690 g/h und die Isobutanolmenge auf 3.400 ml/h gesteigert.

Dies entspricht einer Belastung des Katalysators von 0,192 bis 0,914 g TMC/g Katalysator und Stunde. Im Reaktoraustrag kann gaschromatographisch kein TMC (> 0,1 Gew.-%) nachgewiesen werden.

Anschliessend wird die Hydrierung bei konstantem Zulauf (160 g TMC/h, 790 ml Isobutanol/h) betrieben. Insgesamt werden 71,42 kg TMC zugefahren. Die Reinheit des TMC liegt im Bereich bei 99,25 Gew.-%. Der Reaktoraustrag wird eingedampft. Es werden 72,12 kg 2,3,6-Trimethylhydrochinon in einer Reinheit von 99,6% erhalten. Die Selektivität der Umwandlung von TMC zu Trimethylhydrochinon beträgt 100%. Auch nach 446 Stunden beträgt die Selektivität noch 100%. Eine Aktivitätsabnahme des Katalysators wurde nicht festgestellt.

**Patentansprüche**

1. Kontinuierliches Verfahren zur Herstellung von 2,3,6-Trimethylhydrochinon durch katalytische Hydrierung von 2,3,6-Trimethylchinon mit Wasserstoff in Gegenwart eines Lösungsmittels, dadurch gekennzeichnet, dass die Umsetzung an einem Platin/Aluminiumoxid-Katalysator durchgeführt wird, wobei der Aluminiumoxidträger eine Oberfläche nach BET von 1 bis 350 und ein Porenvolumen im Porenradienbereich von 1,8 bis 60.000 nm von 0,35 bis 0,60 cm³/g besitzt, wobei mindestens 60% dieses Porenvolumens von Poren mit einem Radius im Bereich von 2 bis 200 nm gebildet wird.

2. Verfahren nach Anspruch 1, dadurch gekennzeichnet, dass man die Umsetzung bei einem Wasserstoffpartialdruck von 0,5 bis 5 bar und Temperaturen zwischen 80 und 120°C durchführt.

3. Verfahren nach Anspruch 1 und 2, dadurch gekennzeichnet, dass man das Lösungsmittel durch Abdampfen vom Hydrierprodukt abtrennt und in den Prozess zurückführt.

**Claims**

1. A continuous process for the preparation of 2,3,6-trimethylhydroquinone by catalytic hydrogenation of 2,3,6-trimethylquinone with hydrogen in the presence of a solvent, wherein the reaction is carried out over a platinum/alumina catalyst, the alumina carrier having a BET surface area of from 1 to 350 m²/g and a pore volume of from 0.35 to 0.60 cm³/g in the pore radius range from 1.8 to 60,000 nm, not less than 60% of this pore volume being constituted by pores having a radius of from 2 to 200 nm.

2. A process as claimed in claim 1, wherein the reaction is carried out under a hydrogen partial pressure of from 0.5 to 5 bar and at from 80 to 120°C.

3. A process as claimed in claims 1 and 2, wherein the solvent is evaporated off from the hydrogenation product and recycled to the process.

**Revendications**

1. Procédé continu de préparation de 2,3,6-triméthylhydroquinone par hydrogénation catalytique de 2,3,6-triméthylquinone avec de l'hydrogène, en présence d'un solvant, caractérisé par le fait que la réaction est effectuée sur un catalyseur platine/oxyde d'aluminium, le support oxyde d'aluminium possédant une surface selon BET de 1 à 350 et un volume de pores, dans les limites de rayons de pores de 1,8 à 60 000 nm, de 0,35 à 0,60 cm³/g, au moins 60% de ce volume de pores étant formé de pores d'un rayon allant de 2 à 200 nm.

2. Procédé selon la revendication 1, caractérisé par le fait que l'on effectue la réaction à une pression partielle d'hydrogène de 0,5 à 5 bar et une température comprise entre 80 et 120°C.

3. Procédé selon les revendications 1 et 2, caractérisé par le fait que l'on sépare le solvant par évaporation du produit hydrogéné et qu'on le recycle.